# EUROPÄISCHE PATENTSCHRIFT

(11) **EP 1 184 044 B1**
(45) Veröffentlichungstag und Bekanntmachung des Hinweises auf die Patenterteilung: **26.05.2004**
(21) Anmeldenummer: 00118599.0
(22) Anmeldetag: 28.08.2000
(51) Int. Cl.: A61M 1/06

(54) **Brusthaubeneinsatz**
Breast cup insert
Bonnet d'un sein

(43) Veröffentlichungstag der Anmeldung: 06.03.2002
(73) Patentinhaber: Medela AG, 6340 Baar (CH)
(72) Erfinder: Greter, Andy, 6312 Steinhausen (CH); Silver, Brian, Cary, IL 60013 (US)
(74) Vertreter: Clerc, Natalia

(56) Entgegenhaltungen:
- WO-A-00/47068
- US-A- 4 263 912
- US-A- 4 607 596
- US-A- 5 149 336
- US-A- 5 776 177

## Beschreibung

Die vorliegende Erfindung betrifft einen Brusthaubeneinsatz für Brustpumpen, mit einem im wesentlichen kegelstumpfförmigen Rahmen mit einer grossen hinteren Oeffnung und einer kleinen vorderen Oeffnung, welche zum Anliegen an die Areola einer weiblichen Brust vorgesehen ist.

Brusthaubeneinsätze der vorstehend beschriebenen Art sind seit einigen Jahren bekannt und dienen einer Anpassung der Haube an unterschiedliche Brustgrössen sowie einer verbesserten Stimulation der Muttermilchleistung.

Aus US-A-4 263 912 ist ein Brusthaubeneinsatz bekannt, der einen Rahmen mit einer grossen hinteren Öffnung une einer kleinen vorderen Öffnung aufweist, wobei die Rahmenwand als Doppelwand ausgebildet ist, die innenliegende Wand elastisch deformierbar ist und die Doppelwand ein deformierbares Medium enthält.

Aufgabe der vorliegenden Erfindung war es, die Brusthaubeneinsätze durch besondere Ausgestaltung bzw. besonderen Aufbau des Einsatzes noch weiter zu optimieren und deren Verwendung noch angenehmer zu gestalten.

Diese Aufgabe wurde bei einem Brusthaubeneinsatz der eingangs definierten Art erfindungsgemäss durch die Merkmale des Anspruchs 1 gelöst .

Die Wirksamkeit und der Anwendungskomfort sind beim Erfindungsgegenstand im Vergleich zum Stand der Technik noch wesentlich gesteigert.

Besondere Ausführungsformen des Erfindungsgegenstandes sind in den abhängigen Ansprüchen definiert.

Die Erfindung wird nachstehend anhand von in der Zeichnung dargestellten Ausführungsbeispielen noch etwas näher erläutert.

Es zeigt:
- Fig. 1: einen Vertikalschnitt durch eine erste Ausführungsform eines erfindungsgemässen Einsatzes;
- Fig. 2: eine Variante, ebenfalls im Vertikalschnitt, und
- Fig. 3: eine weitere Variante mit Zusatzfolie.

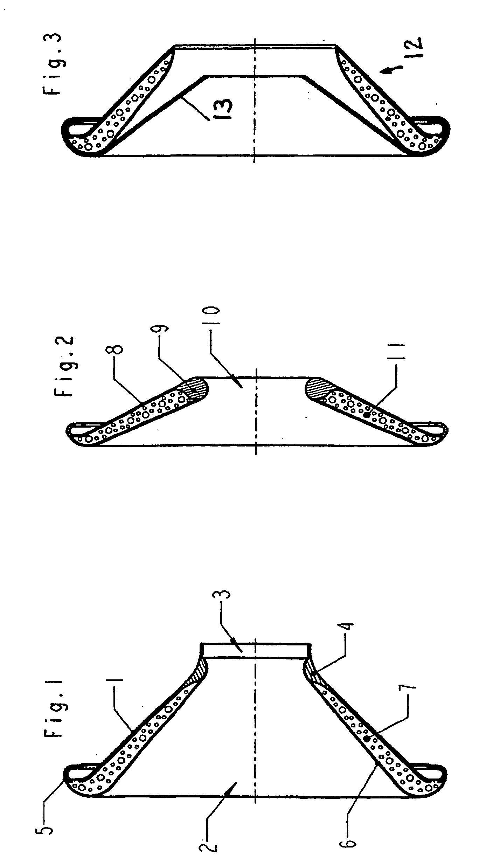

Figur 1 der Zeichnung zeigt einen kegelstumpfförmigen Brusthaubeneinsatz mit einem Rahmen 1 (aus Kunststoff oder gummielastischem Material) mit einer hinteren grossen Oeffnung 2 und einer vorderen kleinen Oeffnung 3. Auf der radial inneren Seite der kleinen Oeffnung 3 ist eine Erhöhung (Verdickung) 4 vorgesehen, welche der Auflage an die Areola einer weiblichen Brust dient (zwecks Stimulation). Zwischen dieser Erhöhung 4 und dem Rand 5 der grossen Oeffnung 2 verläuft eine elastische Innenwand 6 (z.B. PU-Folie) und bildet mit dem Rahmen 1 eine Kammer 7, welche ein deformierbares Medium (Gas, z.B. Luft; eine verdickte Flüssigkeit; ein Gel; Silikon) enthält. Diese Massnahme trägt wesentlich zur Erhöhung des Komforts bei der Anwendung bei.

Figur 2 zeigt eine Variante eines Brusthaubeneinsatzes, mit gegenüber der Ausführungsform nach Figur 1 leicht abweichender Konstruktion des Rahmens 8. Dieser besteht aus einem kegelstumpfförmigen Hohlprofil aus elastischem Material, wobei auch hier die Erhöhung 9 an der kleinen Oeffnung 10 und die Füllung der Kammer 11 vorhanden ist.

Figur 3 zeigt eine weitere Variante eines Brusthaubeneinsatzes 12 mit im wesentlichen gleichem Aufbau wie die Ausführungsformen nach Figur 1 oder 2, welche an Stelle der an der kleinen Rahmenöffnung vorgesehenen Erhöhung (4 bzw. 9) eine elastische Zentrierfolie 13 aufweist (Silikonfolie), welche ebenfalls der "Zentrierung" der Brust und Stimulation dient. Zentrierfolie 13 erlaubt eine optimale Positionierung der Brustwarze und vermag ein unerwünschtes Herunterlaufen von Milch nach Entfernen der Haube zu verhindern (Auffangen der Milch im Raum zwischen dem Einsatzrahmen 1, 8 und der Zentrierfolie 13.

## Patentansprüche

1. Brusthaubeneinsatz für Brustpumpen, mit einem im wesentlichen kegelstumpfförmigen Rahmen (1, 8) mit einer grossen hinteren Oeffnung (2) fund einer kleinen vorderen Oeffnung (3, 10), welche zum Anliegen an die Areola einer weiblichen Brust vorgesehen ist, wobei die kegelförmige Rahmenwand unter Bildung einer geschlossenen Kammer (7, 11) als Doppelwand ausgebildet ist, wobei zumindest die innenliegende Wand (6) elastisch deformierbar ist und die Kammer ein deformierbares Medium enthält.

2. Brusthaubeneinsatz nach Anspruch 1, **dadurch gekennzeichnet, dass** das Füllmedium der Kammer (7) ein Gas, wie z.B. Luft, eine vorzugsweise verdickte Flüssigkeit, ein Gel oder Silikon ist.

3. Brusthaubeneinsatz nach Anspruch 1 oder 2, **dadurch gekennzeichnet, dass** auf der innenliegenden Seite der kleineren Rahmenöffnung (3, 10) eine radial nach innen vorstehende umlaufende Erhöhung (4, 9) vorgesehen ist.

4. Brusthaubeneinsatz nach Anspruch 3, **dadurch gekennzeichnet, dass** die Erhöhung (4, 9) als deformierbare Kammer, vorzugsweise ein Füllmedium, wie z.B. Silikon, enthaltend ausgebildet ist.

5. Brusthaubeneinsatz nach Anspruch 3 oder 4, **dadurch gekennzeichnet, dass** sich die elastisch deformierbare innenliegende Wand (6) des Rahmens zwischen dem Rand der grösseren Oeffnung und dem innenliegenden Rand der Erhöhung an der kleineren Oeffnung erstreckt.

6. Brusthaubeneinsatz nach Anspruch 1 oder 2, **dadurch gekennzeichnet, dass** im Innern des Einsatzes eine elastische Zentrierfolie (13) angeordnet ist, welche abnehmbar oder fest mit dem Rahmen verbunden ist.

## Claims

1. Breast cover insert for breast pumps, with a substantially truncated cone shaped frame (1, 8) with a large rear opening (2) and a small front opening (3, 10) which is adapted to abut the areola of a female breast, wherein the truncated cone shaped frame wall is designed by forming a closed chamber (7, 11) as double wall, whereby at least the inner wall (6) is elastically deformable and the chamber contains a deformable medium.

2. Breast cover insert according to claim 1, **characterized in that** the filling medium of the chamber (7) is a gas, such as e.g. air, a preferably thickened liquid, a gel or silicon.

3. Breast cover insert according to claim 1 or 2, **characterized in that** radially inwards projecting circumferential heightening (4, 9) is foreseen at the inner side of the smaller frame opening (3, 10).

4. Breast cover insert according to claim 3, **characterized in** the heightening (4, 9) is designed as a deformable chamber, containing preferably a filling medium, such as e.g. silicon.

5. Breast cover insert according to claim 3 or 4, **characterized in that** the elastically deformable inner wall (6) of the frame extends between the edge of the larger opening and the inner edge of the heightening at the smaller opening.

6. Breast cover insert according to claim 1 or 2, **characterized in that** a elastic centering foil (13) is located within the insert which is detachably or firmly connected to the frame.

## Revendications

1. Entonnoir pour tire-lait, comprenant un cadre de forme essentiellement tronconique (1, 8) avec une grande ouverture arrière (2) et une petite ouverture avant (3, 10), qui est prévue pour s'appliquer contre l'aréole d'un sein maternel, la paroi du cadre de forme conique étant réalisée sous forme de double paroi formant une chambre fermée (7, 11), au moins la paroi interne (6) étant déformable élastiquement et la chambre contenant un milieu déformable.

2. Entonnoir selon la revendication 1, **caractérisé en ce que** le milieu de remplissage de la chambre (7) est un gaz, par exemple de l'air, un liquide de préférence épaissi, un gel ou de la silicone.

3. Entonnoir selon la revendication 1 ou 2, **caractérisé en ce que** l'on prévoit sur le côté interne de la petite ouverture de cadre (3, 10) un rehaussement (4, 9) périphérique saillant radialement vers l'intérieur.

4. Entonnoir selon la revendication 3, **caractérisé en ce que** le rehaussement (4, 9) est réalisé sous forme de chambre déformable, contenant de préférence un milieu de remplissage comme par exemple de la silicone.

5. Entonnoir selon la revendication 3 ou 4, **caractérisé en ce que** la paroi interne (6) du cadre, déformable élastiquement, s'étend entre le bord de la grande ouverture et le bord interne du rehaussement sur la petite ouverture.

6. Entonnoir selon la revendication 1 ou 2, **caractérisé en ce que** l'on dispose à l'intérieur de l'entonnoir une membrane de centrage élastique (13) qui peut être enlevée ou être connectée fixement au cadre.
